# EUROPEAN PATENT SPECIFICATION

(11) **EP 2 251 436 B1**
(45) Date of publication and mention of the grant of the patent: **21.11.2012**
(21) Application number: 09709058.3
(22) Date of filing: 29.01.2009
(51) Int. Cl.: C12Q 1/68, C12N 15/09

(54) **SIRNA DETECTION METHOD**
SIRNA-NACHWEISVERFAHREN
PROCÉDÉ DE DÉTECTION D'ARNSI

(30) Priority: 04.02.2008 JP 2008024156
(43) Date of publication of application: 17.11.2010
(73) Proprietor: Genecare Research Institute Co., Ltd., Kamakura-shi, Kanagawa 247-0063 (JP)
(72) Inventor: FUTAMI, Kazunobu, Kamakura-shi Kanagawa 247-0063 (JP); FURUICHI, Yasuhiro, Kamakura-shi Kanagawa 247-0063 (JP)
(74) Representative: Bösl, Raphael Konrad
(86) International application number: PCT/JP2009/051405
(87) International publication number: WO 2009/098988

(56) References cited:
- WO-A2-2004/085667
- JP-A- 2006 166 907
- JP-T- 2007 532 100
- FUTAMI K ET AL: "Quantitation of full-size small interfering RNA by tailing with terminal deoxynucleotidyl transferase and reverse transcription-polymerase chain reaction analysis", ANALYTICAL BIOCHEMISTRY, ACADEMIC PRESS INC, NEW YORK, vol. 385, no. 2, 15 February 2009 (2009-02-15), pages 386-388, XP025771497, ISSN: 0003-2697, DOI: DOI:10.1016/J.AB.2008.11.037 [retrieved on 2008-12-06]
- FUTAMI KAZUNOBU ET AL: "Anticancer activity of RecQL1 helicase siRNA in mouse xenograft models", CANCER SCIENCE, JAPANESE CANCER ASSOCIATION, TOKYO, JP, vol. 99, no. 6, 1 June 2008 (2008-06-01), pages 1227-1236, XP002577313, ISSN: 1347-9032, DOI: DOI:10.1111/J.1349-7006.2008.00794.X [retrieved on 2008-04-14]
- RO S ET AL: "A PCR-based method for detection and quantification of small RNAs", BIOCHEMICAL AND BIOPHYSICAL RESEARCH COMMUNICATIONS, ACADEMIC PRESS INC. ORLANDO, FL, US, vol. 351, no. 3, 22 December 2006 (2006-12-22), pages 756-763, XP024925751, ISSN: 0006-291X, DOI: DOI:10.1016/J.BBRC.2006.10.105 [retrieved on 2006-12-22]
- RAYMOND C K ET AL: "SIMPLE, QUANTITATIVE PRIMER-EXTENSION PCR ASSAY FOR DIRECT MONITORING OF MICRORNAS AND SHORT-INTERFERING RNAS", RNA, COLD SPRING HARBOR LABORATORY PRESS, US, vol. 11, no. 11, 1 November 2005 (2005-11-01), pages 1737-1744, XP009060649, ISSN: 1355-8382, DOI: DOI:10.1261/RNA.2148705
- CHEN CAIFU ET AL: "Real-time quantification of microRNAs by stem-loop RT-PCR", NUCLEIC ACIDS RESEARCH, OXFORD UNIVERSITY PRESS, SURREY, GB, vol. 33, no. 20, 1 January 2005 (2005-01-01), page E179, XP002480784, ISSN: 0305-1048, DOI: DOI:10.1093/NAR/GNI178 [retrieved on 2005-11-27]
- KE XI-SONG ET AL: "MicroRNAs: Key participants in gene regulatory networks.", CURRENT OPINION IN CHEMICAL BIOLOGY, vol. 7, no. 4, August 2003 (2003-08), pages 516-523, XP002615443, ISSN: 1367-5931
- AHMED F E: "Role of miRNA in carcinogenesis and biomarker selection: A methodological view", EXPERT REVIEW OF MOLECULAR DIAGNOSTICS, FUTURE DRUGS, LONDON, GB, vol. 7, no. 5, 1 January 2007 (2007-01-01) , pages 569-603, XP009108568, ISSN: 1473-7159, DOI: DOI:10.1586/14737159.7.5.569

### SEQUENCE LISTING

<110> GENECARE RESEARCH INSTITUTE CO.LTD.
<120> siRNA detection method
<130> G3-A0801P
<150> JP 2008-024156
   <151> 2008-02-04
<160> 2
<170> PatentIn version 3.4
<210> 1
   <211> 21
   <212> DNA
   <213> Artificial
<220>
   <223> an artificially synthesized sequence
<220>
   <221> misc_feature
   <223> DNA/RNA
<400> 1
   guucagacca cuucagcuut t 21
<210> 2
   <211> 21
   <212> DNA
   <213> Artificial
<220>
   <223> an artificially synthesized sequence
<220>
   <221> misc_feature
   <223> DNA/RNA
<400> 2
   cuuacgcuga guacuucgat t 21

## Description

### Technical Field

The present invention relates to methods for detecting siRNAs.

### Background Art

RNA interference (abbreviated as RNAi) is a biological phenomenon triggered by small-molecule double-stranded RNA (small interfering RNA: abbreviated as siRNA). Nucleotide-sequence-specific degradation of messenger RNA (mRNA) suppresses production of a protein encoded by that mRNA, and as a result suppresses the expression of specific genes. This biological phenomenon which takes place in the cytoplasm of a cell is widely used as a tool for molecular biological studies and for the purpose of drug discovery research and such which screen for drug candidate proteins. Double-stranded RNAs artificially sent into cells can suppress the copy number of strictly specified proteins, even at a low concentration. Thus drug development which attempts to utilize synthesized double-stranded RNAs as nucleic acid pharmaceuticals is being promoted. For this purpose, short siRNAs consisting of 21 mers to 25 mers exhibit sufficient effects, but due to technical reasons relating to siRNA synthesis, as well as economic reasons, the shortest 21-mer siRNAs are often selected as pharmaceutical candidates. In fact, if the nucleotide sequences are appropriately selected, these siRNAs can suppress the production of specific proteins in cells at low, nanomolar-level concentrations, and are therefore predicted to become pharmaceuticals with few side effects, and anticipated to become the next-generation pharmaceuticals following antibody pharmaceuticals. While antibody pharmaceuticals exhibit pharmacological effects outside cells, siRNA pharmaceuticals act only in a restricted place, namely the cytoplasm inside cells, and thus, safety can also be expected, in terms of not making unnecessary contact with genetic DNA in the nucleoplasm. Furthermore, siRNA pharmaceuticals have enabled development of pharmaceuticals that had been difficult with conventional low-molecular-weight pharmaceuticals, such as siRNA pharmaceuticals targeting proteins that do not indicate biochemical activities that serve as an indicator for screening, and siRNA pharmaceuticals with high selectivity towards structurally very similar proteins. Thus siRNA pharmaceuticals are pointed out to be advantageous in expanding the range of drug discovery.

Use of an siRNA carrier synthesized and labeled with a radioisotope is known as one of the conventionally-used methods for quantifying minute amounts of siRNA, and this is not necessarily impossible. However, it has many problems such as those indicated below, and is not a sensible method. Specifically,
(1) there are restrictions on the locations, and machines/equipments for carrying out the synthesis;
(2) there are restrictions on the manufacturer carrying out the synthesis;
(3) nucleotides containing a radioisotope are expensive;
(4) the purity of nucleotides containing a radioisotope is often questionable and as a result, the purity of the synthesized siRNA is not truly reliable;
(5) there are restrictions on the locations, instruments, human resources, and such for purification of minute amounts of radioisotope-containing siRNA, and whether an siRNA carrier with truly high purity can be obtained is uncertain; and
(6) even if radioisotope-containing siRNA can be produced, its application will remain to specific locations and for use on laboratory animals, and the most important point is that, it cannot be administered to humans;
and so there are countless numbers of disadvantages. For this reason, development of siRNA quantification techniques that do not use radioisotopes is strongly desired.

In addition to the above-mentioned siRNAs, recently active RNA research that is attracting attention features miRNAs having the function of regulating translation of proteins by binding to the 3' UTR of mRNAs. Similarly to siRNA, miRNA is known to exist as a single strand as a result of conversion by RISC, and becomes a mature form having a chain length of 22 nucleotides. Since miRNA is entirely constituted by RNA, a method developed for detecting miRNA comprises carrying out polyadenylation and such on the RNA, performing cDNA synthesis by reverse transcription reaction using oligodT which anneals to the produced polyA portion as a primer, and subsequently amplifying the miRNA by performing PCR.

QIAGEN has developed a method as described below for miRNA detection (miScript system). The method involves
(1) adding a poly(A) tail using polyA polymerase on a small noncoding RNA containing miRNA extracted from a living body;
(2) synthesizing cDNA by performing a reverse transcription reaction using a total RNA containing polyA as a template and poly(T) containing a universal tag sequence; and
(3) performing realtime PCR between the universal tag sequence and an miRNA-specific primer (SYBR green method). Quantification becomes possible from the difference with respect to the amplification efficiency of a reference gene.

In addition, the TaqMan MicroRNA Assay of ABI is a technique in which
(1) cDNA is synthesized by hybridizing an RT-primer having higher-order (loop) structure optimized for each miRNA to the miRNA terminus; and
(2) subsequently PCR is performed between the specific primer and the sequence in the loop structure, and quantification is carried out using fluorescence liberated from an internal fluorescence-labeled probe (TaqMan probe).

The methods of both companies utilize the characteristic that miRNA is a pure RNA. In the technique of QLAGEN, since tailing is carried out using an RNA-dependent poly(A) polymerase, the template must be RNA. In the technique of ABI, higher order structure primers designed specifically for each miRNA are necessary. This is a technique that can be used only because the types of miRNAs are limited in a living body. On the other hand, in the case of siRNAs that are chemically synthesized and used by introduction from the outside, many of them have, for example, chimeric structures with d(TT) DNA overhang at the ends, and the sequences used are also diverse. Therefore, both of the above-mentioned techniques cannot be applied.

Although it can be predicted from many previous studies that siRNAs have excellent properties as pharmaceuticals, siRNAs are completely novel pharmaceutical materials which mankind has never encountered before. Thus, there are problems that conventional techniques are insufficient for the elucidation of pharmacokinetics or such necessary in the process of developing pharmaceuticals. That is, there is still no effective method for accurately, rapidly, and economically measuring the *in vivo* kinetics of minute amounts of siRNA, such as accumulation in tissues, change in blood concentration, and metabolism over time, and this is one of the great hurdles in the development of siRNA pharmaceuticals.

This hurdle must be overcome in order to develop siRNA pharmaceuticals having a number of excellent properties, and therefore development of siRNA measurement techniques has become a major objective. Many clinical studies of siRNA pharmaceuticals will be carried out throughout the world in the future, and there is a pressing demand for methods that can sensitively follow the administered siRNAs with regard to their pharmacokinetics in the blood, organs, and tissues, and their behavior in cells.

Prior art documents are indicated below.
Patent Document 1: WO 2005/021800 A2
Patent Document 2: WO 2005/098029 A2
Patent Document 3: WO 2004/085667 A2
Non-patent Document 1: Lee DY. et al., MicroRNA-378 promotes cell survival, tumor growth, and angiogenesis by targeting SuFu and Fus-1 expression. Proc. Natal Acad. Sci. U.S.A., 2007 Dec; 51: 20350-5
Non-patent Document 2: Chen C, et al., Real-time quantification of microRNAs by stem-loop RT-PCR. Nucleic Acids Res., 2005 Nov 27; 33(20): e179
Non-patent Document 3: Ro S, Park C, Jin J, Sanders KM, Yan W., A PCR-based method for detection and quantification of small RNAs. Biochem Biophys Res Commun. 2006 Dec 22; 351(3):756-63
Non-patent Document 4: Overhoff M, Wunsche W, Sczakiel G., Quantitative detection of siRNA and single-stranded oligonucleotides: relationship between uptake and biological activity of siRNA. Nucleic Acids Res. 2004 Dec 2; 32(21): e170
Non-patent Document 5: Raymond CK, Roberts BS, Garrett-Engele P, Lim LP, Johnson JM., Simple, quantitative primer-extension PCR assay for direct monitoring of microRNAs and short-interfering RNAs. RNA. 2005 Nov; 11(11): 1737-44
Non-patent Document 6: Chen C, Ridzon DA, Broomer AJ, Zhou Z, Lee DH, Nguyen JT, Barbisin M, Xu NL, Mahuvakar VR, Andersen MR, Lao KQ, Livak KJ, Guegler KJ., Real-time quantification of microRNAs by stem-loop RT-PCR. Nucleic Acids Res. 2005 Nov 27; 33(20): e179

### Disclosure of the Invention

### [Problems to be Solved by the Invention]

An objective of the present invention is to provide methods for efficiently detecting chemically synthesized siRNAs, such as those with a chimeric structure containing DNA overhangs at the ends.

### [Means for Solving the Problems]

The present inventors carried out dedicated studies to solve the above-mentioned objective. Envisaging detection of siRNAs with a chimeric structure having d(TT) DNA overhangs at the ends, the present inventors performed reactions in the following order and succeeded in detecting and quantifying minute amounts of siRNAs.
(1) First, by focusing on the general structure of chemically synthesized siRNAs, a polydG tail is added to the d(TT) overhangs of siRNAs using terminal deoxynucleotidyl transferase with dGTP as the substrate. This operation has a purpose of differentiation from poly rA tailing of other mRNAs and tagging. Furthermore, since the chain length of the tail cannot be controlled with polydC and polydT, polydG is desirable.
(2) Next, a primer for a polydC sequence added with a tag sequence is annealed and cDNA is synthesized. Since the tag sequence is used as a priming site when performing PCR according to the following operation, its length is desirably comparable to that of a common primer.
(3) Quantitative PCR is performed between a primer carrying the same sequence as the tag sequence, and a primer containing the same sequence (except U is replaced with T) as the siRNA sequence to be detected. A known technique can be used for the quantitative PCR. For example, a system utilizing commercially available SYBR Green (intercalates only into dsDNA) can be used.
(4) From a calibration curve produced using known amounts of short-chain dsDNA, the amount of siRNA of Interest can be determined.
(5) When using the SYBR green detection system, the reactions up to the above-mentioned (2) are carried out in the same tube; therefore, a known amount of an internal standard (siRNA) can be added in advance in the process of RNA extraction or in the process of cDNA synthesis, and the amount of siRNA can be corrected based on the value.

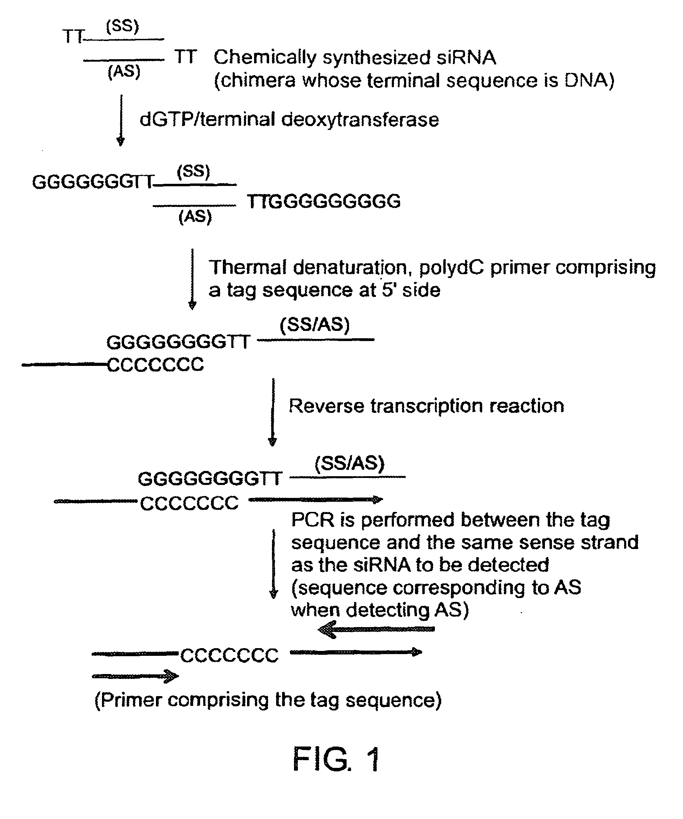

The above-mentioned reaction scheme is shown in Fig. 1.

As described above, the present inventors succeeded in developing methods for efficiently detecting siRNAs containing DNA overhangs at the ends, particularly chemically synthesized siRNAs, and completed the present invention.

The present inventors discovered that siRNAs of interest can be detected efficiently by using guanine (dG) as the base added by terminal deoxytransferase or such to the bases constituting the overhangs of siRNAs to be detected. This discovery is an advantageous effect that cannot be easily reached from conventional findings, even by those skilled in the art.

The present invention relates to methods for efficiently detecting siRNA containing DNA overhangs at the 3' ends, for example, chemically synthesized siRNAs, and more specifically the present invention provides the following:
[1] a method for detecting an siRNA comprising a DNA overhang at the 3' end, which comprises the steps of:
   (a) adding polydeoxyguanine (polydG) to the 3'-terminal overhang of an siRNA;
   (b) synthesizing a single-stranded DNA by performing a reverse transcription reaction using the molecule produced by the previous step as a template and a polydeoxycytosine (polydC) primer comprising a tag sequence at the 5' side;
   (c) performing PCR using the single-stranded DNA as a template, a primer that anneals to the complementary strand of the tag sequence, and a primer that anneals to one of the strands of the siRNA; and
   (d) detecting the PCR product;
[2] a method for selectively amplifying a sequence constituting an siRNA comprising a DNA overhang at the 3' end, which comprises the steps of:
   (a) adding polydeoxyguanine (polydG) to the 3'-terminal overhang of an siRNA;
   (b) synthesizing a single-stranded DNA by performing a reverse transcription reaction using the molecule produced by the previous step as a template and a polydeoxycytosine (polydC) primer comprising a tag sequence at the 5' side; and
   (c) performing PCR using the single-stranded DNA as a template, a primer that anneals to the complementary strand of the tag sequence, and a primer that anneals to one of the strands of the siRNA;
[3] the method of [1] or [2], wherein the addition of polydeoxyguanine (polydG) is carried out by terminal deoxytransferase; and
[4] a reagent for detecting siRNA comprising a DNA overhang at the 3' end, which comprises a primer according to [1](b) or (c) (for example, a polydeoxycytosine (polydC) primer comprising a tag sequence at the 5' side, a primer that anneals to the complementary strand of the tag sequence, and a primer that anneals to one of the strands of an siRNA).

### Brief Description of the Drawings

Fig. 1 schematically shows an example of the siRNA detection method of the present invention. Quantitative PCR is possible by producing a primer dependent on the sequence to be detected. Furthermore, quantitative PCR using SYBR Green is also possible.
Fig. 2 depicts the amplification curves derived from siRNA with known amounts for the standard curve. (A) Starting from the left, the curves correspond to 1500, 1000, 500, 100, 50, 20, 10, and 5 fmol. The calibration curve is shown in (B).
Fig. 3 depicts the amplification curves obtained by measuring actual samples with the method of the present invention.

### Mode for Carrying Out the Invention

The present invention relates to methods for detecting siRNAs present in samples (herein, they may be referred to as methods of the present invention").

The siRNAs detected by the methods of the present invention are usually siRNAs containing DNA overhangs at the 3' ends. The bases constituting the terminal overhangs of these siRNA are usually DNA. The type of base of the DNA is not particularly limited, and siRNAs with a structure in which any bases are overhanging can be detected using the methods of the present invention.

Generally, chemically synthesized siRNAs often have structures in which (deoxy) thymines (dT) are overhanging at the 3' ends, and in the methods of the present invention, for example, siRNAs with a structure in which (deoxy) thymine residues are overhanging at the 3' ends can be favorably detected. Furthermore, the length of the bases constituting an overhang is not particularly limited, but is usually one- to two-bases long, and is preferably two-bases long. Preferred methods in the present invention are methods for detecting siRNAs with a structure in which (deoxy) thymine forms two-base overhangs at the 3' ends.

A preferred embodiment of the methods of the present invention includes methods of detecting an siRNA containing DNA overhangs at the 3' ends, which comprise the steps of:
(a) adding polydeoxyguanine (polydG) to the overhangs (DNA) of an siRNA;
(b) synthesizing a single-stranded DNA by performing a reverse transcription reaction using the molecule produced by the previous step as a template and polydeoxycytosine (polydC) primer containing a tag sequence at the 5' side;
(c) performing PCR using the single-stranded DNA as a template, a primer that anneals to the complementary strand of the tag sequence, and a primer that anneals to one of the strands of the siRNA; and
(d) detecting the PCR product.

A preferred embodiment of the methods of the present invention is shown schematically in Fig. 1. However, the present invention is not necessarily limited to the method shown in Fig. 1.

In the above-mentioned step (a), the length of the polydG added to the bases constituting the overhangs is not particularly limited, but is usually approximately 10- to 20-mer long.

Addition of polydG can be carried out using, for example, terminal deoxytransferase and dGTP. Enzymes that carry out the reaction of the addition are not particularly limited as long as they have an activity to add dGTP to the ends of double-stranded nucleic acids containing overhangs.

In the present invention, usually, siRNAs containing DNA overhangs at the 3' ends are the target of detection, but for example, siRNAs without overhangs can also be detected by the methods of this application by adding a DNA or ribo-polyG to the 3' ends of such siRNAs.

For addition of DNAs to the blunt-ended siRNAs without overhangs, a DNA polymerase that acts in a template-independent manner can be used, for example. Furthermore, the methods of the present invention can be carried out upon adding ribo-polyG to siRNAs with blunt ends. In such a case, ribo-polyG can be added, for example, by using rGDP and polynucleotide phosphorylase (PNPase). Alternatively, primer-dependent PNPase may be used to add ribo-polyG. For terminal addition of very short nucleotides, Taq polymerase can be used.

The above-mentioned step (a) has effects of tagging and distinguishing from polydA tailing of other mRNAs. Since the length of the chain used for tailing is difficult to regulate with polydC and polydT, polydG is desirable.

PolydG synthesized in advance (for example, approximately 10 to 20 mers in length) can be added to the ends of double-stranded nucleic acids containing overhangs.

In the above-mentioned step (b), the chain length of the tag sequence and the order of the bases are not particularly limited, but since this sequence is used as a primer in the above-mentioned step (c), it preferably has a length that can be usually used as a primer for PCR. Examples include lengths such as 5 to 30 mers and preferably approximately 10 to 20 mers.

The polydC region of the above-mentioned "polydeoxycytosine (polydC) primer containing a tag sequence at the 5' side" has homology with the polydG region added in the above-mentioned step (a). Thus, it anneals to the dG region and functions as a primer for the reverse transcription reaction.

The reverse transcription reaction of the above-mentioned step (b) can be carried out appropriately by common methods using a reverse transcriptase by those skilled in the art. Commercially available reverse transcriptases generally used by those skilled in the art can be used appropriately, and their type is not particularly limited. Commercially available reagents, kits, or such for the reverse transcription reaction can also be used appropriately.

The siRNAs to which polydGs were added by the reverse transcription reaction of step (b) are converted to single-stranded DNAs. This step synthesizes a single-stranded DNA with a structure in which a polydC containing a tag sequence at the 5' side and a cDNA corresponding to one of the chains of the siRNA are bound. This single-stranded DNA is further subjected to reactions for amplification of this DNA.

Step (c) of the present invention is a step of using a single-stranded DNA synthesized in step (b) as a template to amplify the sequence corresponding to this DNA. The technique used in the above-mentioned step (c) is not particularly limited as long as it is a technique for amplifying the single-stranded DNA synthesized in step (b), but usually, a polymerase chain reaction (PCR) is carried out using the single-stranded DNA as a template. This PCR is a known technique, and various commercially available reagents, kits, or such may be used appropriately.

This PCR is usually conducted using a set of primers that anneal to the terminal regions of the DNA which will serve as the template. This can be carried out without problem even when the DNA which will serve as the template is single-stranded. Primers for amplifying a single-stranded DNA can be easily designed and synthesized by those skilled in the art based on sequence information of the single-stranded DNA that serves as the template.

In the method of the present invention, a PCR amplified product specific to the siRNA of interest is detected using primers that anneal to one of the strands (antisense strand or sense strand) of the siRNA to be detected. When the PCR product is detected, the siRNA of interest is determined to be present in the test sample.

Primers used in the above-mentioned step (c) include, for example, (1) a primer containing a region corresponding to the above-mentioned tag sequence, and (2) a primer that anneals to a region corresponding to the siRNA in the single-stranded DNA (a region corresponding to the cDNA of one of the strands of an siRNA). For example, PCR is performed between the above-mentioned tag sequence and the sense strand of the siRNA strands to be detected.

The primer of the above-mentioned (1) is usually a primer containing a sequence produced by removing polydC from "polydC primer containing a tag sequence at the 5' side" used in the above-mentioned step (b), but the primer length can be made shorter or longer as one thinks suitable. Furthermore, the primer of the above-mentioned (1) can be expressed as a primer that anneals to the complementary strand of the tag sequence. The above-mentioned primers of the present invention do not necessarily have to completely match the tag sequence itself, as long as they anneal to the complementary strand of the tag sequence.

The primer of the above-mentioned (2) is, for example, usually a sequence corresponding to the antisense strand (AS) (except that A is dA, G is dG, C is dC, and U is dT) when one wants to detect the sequence corresponding to the antisense (AS) strand in the siRNA, and usually a sequence corresponding to the sense strand when one wants to detect the sequence corresponding to the sense strand in the siRNA.

Furthermore, in the above-mentioned step (c), quantitative PCR can be carried out. A known technique can be used for the quantitative PCR. For example, a system utilizing commercially available SYBR Green (intercalates only into dsDNA) can be used.

In step (d), products amplified by step (c) (PCR products) are detected. PCR products are usually double-stranded DNA, and they can be detected by known methods such as electrophoresis. Commercially available reagents, kits, and such can be used appropriately to detect PCR products.

Furthermore, the amount of an siRNA of interest can be determined from a calibration curve produced using known amounts of a short-chain dsDNA. That is, an siRNA of interest can be quantitatively measured by the methods of the present invention. For example, by using as a control an siRNA whose amount has been determined in advance, the amount of siRNA of interest can be measured.

For example, when a SYBR green detection system is used, reactions are carried out in the same tube; therefore, a known amount of an internal standard (siRNA) is added in advance in the process of RNA extraction or in the process of cDNA synthesis, and the amount of siRNA can be corrected appropriately based on the value.

Furthermore, the present invention provides methods for selectively amplifying siRNAs containing DNA overhangs at the 3' ends. Usually, chemically synthesized siRNAs are, for example, siRNAs containing overhangs such as dTT in many cases; therefore, chemically synthesized siRNAs (nucleotide sequences constituting the siRNAs) can be selectively amplified according to the methods of the present invention.

A preferred embodiment of the above-mentioned methods of the present invention includes methods of selectively amplifying a sequence constituting an siRNA containing DNA overhangs at the 3' ends, which comprises the steps of:
(a) adding polydeoxyguanine (polydG) to the overhangs (DNA) of an siRNA;
(b) synthesizing a single-stranded DNA by performing a reverse transcription reaction using the molecule produced by the previous step as a template and a polydeoxycytosine (polydC) primer containing a tag sequence at the 5' side; and
(c) performing PCR using the single-stranded DNA as a template, a primer that anneals to the complementary strand of the tag sequence, and a primer that anneals to one of the strands of the siRNA.

Furthermore, primers, enzymes, and such used for the methods of the present invention are also included in the present invention. That is, the present invention provides reagents for detecting siRNAs containing DNA overhangs at the 3' ends, which include a primer of the above-mentioned (1) or (2), terminal deoxytransferase, or reverse transcriptase as an active ingredient.

Furthermore, the present invention provides a kit for siRNA detection which is produced by combining multiple substances selected from the group consisting of a primer of the above-mentioned (1) or (2), terminal deoxytransferase, and reverse transcriptase.

The pharmacokinetics of siRNAs administered into individuals can be examined (monitored) by the methods of the present invention. Since siRNAs are short duplexes, which are 20 mers or so, and since the concentration distributed in the blood and in the organs is low (nM level), preferred detection methods did not exist in the past. The methods of the present invention can qualitatively or quantitatively measure the concentrations of siRNAs administered into individuals and distributed in the blood or the organs.

A preferred embodiment of the methods of the present invention relates to methods of monitoring the pharmacokinetics of an siRNA administered to an individual, which comprises the steps of:
(a) obtaining a test sample from a biological site (blood, tissues, etc.) where the amount of existing siRNA is to be measured; and
(b) detecting an siRNA of interest in the test sample by the method of the present invention.

All prior art documents cited in the specification are incorporated herein by reference.

### Examples

Herein below, the present invention will be specifically described with reference to Examples. However, the technical scope of the present invention is not to be construed as being limited to these Examples.

### [Example 1] Measurement of siRNA

The concentration of GL3-siRNA (5'-CUUACGCUGAGUACUUCGAdTdT-3'/SEQ ID NO: 2) introduced into cells was measured using RecQL1-siRNA (5'-GUUCAGACCACUUCAGCUUdTdT -3'/SEQ ID NO: 1) as the internal standard. 50 pmol of GL3-siRNA was transfected (introduced) into A549 cells, the cells were sampled over time, and total RNA was extracted. The miRNAeasy Mini kit from QIAGEN was used for the total RNA extraction, and short-chain RNAs (up to 18 mers) were collected.

A series of reactions were performed and quantitative PCR was carried out. A calibration curve was obtained using known amounts of siRNA, and corrections were made using known amounts of RecQL1-siRNA which was mixed during the operation.

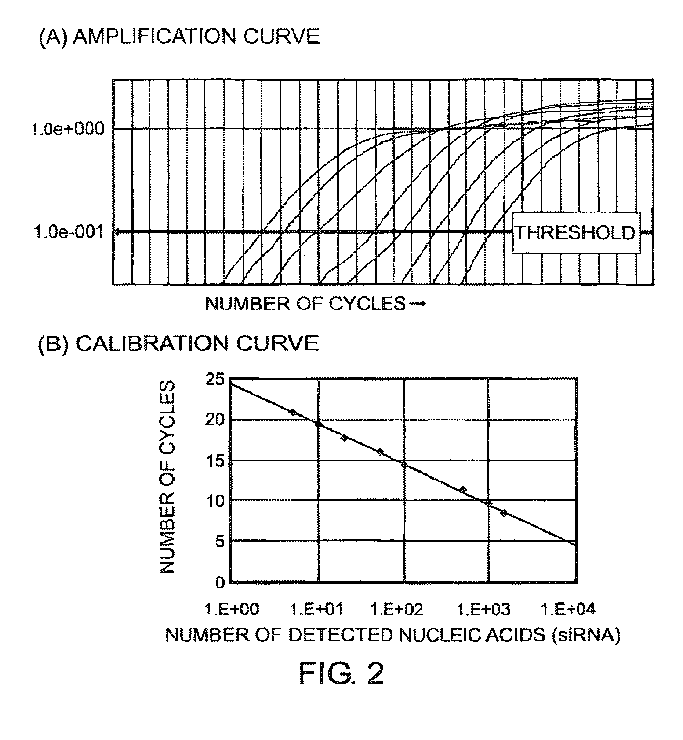

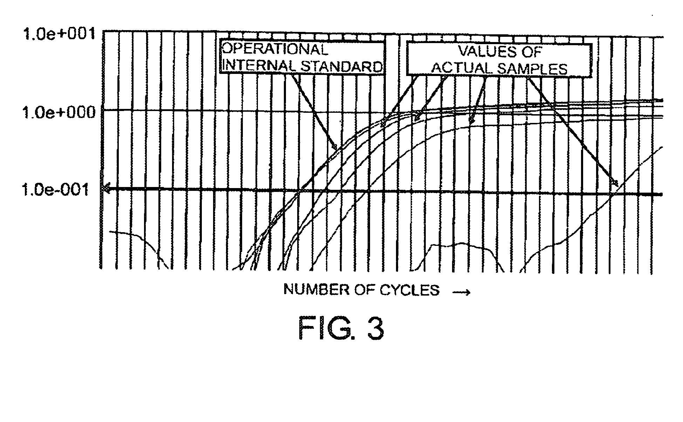

Amplification curves derived from known amounts of siRNA are shown in Fig. 2 as the calibration curve. The results showed that 5 femtomoles to 1.5 picomoles of siRNA can be measured. This measurement range was equivalent to that of an ordinary quantitative RT-PCR. Data obtained from measurements of actual samples are shown in Fig. 3.

When corrected using the quantities of siRNA added to an operational standard, the following was obtained.

**Table 1**

| | | | | | | | | | |
|---|---|---|---|---|---|---|---|---|---|
| 0 | 6 | 12 | 24 | 48 | 72 | 96 | 120 | 144 | hours |
| 0 | 12.6 | 9.9 | 5 | 4.8 | 3.4 | 2.7 | 1.2 | 1.0 | pmol |

Similarly, the concentration of RecQL1-siRNA
(5'-GUUCAGACCACUUCAGCUUdTdT -3'/SEQ ID NO: 1) administered to mice was determined using GL3-siRNA (5'-CUUACGCUGAGUACUUCGAdTdT-3'/SEQ ID NO: 2) as the internal standard. After systemic administration of 50 µg of siRNA to mice, blood was sampled over time, and total RNA was extracted. The miRNAeasy Mini kit from QIAGEN was used for the total RNA extraction, and short-chain RNAs (up to 18 mers) were collected.

A series of reactions were performed and quantitative PCR was carried out. A calibration curve was obtained using known amounts of siRNA, and corrections were made with known amounts of GL3-siRNA which was mixed during the operation. The results are shown in Table 2.

**Table 2**

| Blood | 0 min | 5 min | 15 min | 30 min | 1 hr | 3 hr | 6 hr | 12 hr | 24 hr | 48 hr |
|---|---|---|---|---|---|---|---|---|---|---|
| Ave. (µg) | 50.0 | 9.7 | 7.1 | 5.9 | 4.5 | 0.7 | 0 | 0 | 0 | 0 |
| SD | 0 | 0.3 | 0.3 | 02 | 0.5 | 0.1 | 0.1 | 0 | 0 | 0 |

### Industrial Applicability

The methods of the present invention have the features of converting short double-stranded RNAs (for example, 21 mer siRNAs), which were conventionally considered to be difficult to convert to cDNAs using a reverse transcriptase, into DNAs using various novel ideas that utilize characteristics of siRNAs, and enabling nucleotide sequence-specific measurement and quantification.

These methods have no particular limitations, but for example, they may be used favorably in quantification of synthetic chimeric siRNAs having a structure in which two bases of deoxy TT are overhanging at the 3' ends.

Addition reaction of polydG using dGTP and terminal deoxytransferase is only possible with 3'dTT having a form with DNA overhangs, and this is the characteristic of the present methods in which only siRNAs having the full length are quantified and detected. This means that when siRNAs are extracted from biological samples, RNAs and DNAs which may be contained in the sample will not be taken into quantification operation non-specifically, and this can be said to be an advantage of the present methods.

Furthermore, regulation of the addition reaction to the 3' end was difficult with the combination of a nucleotide 3-phosphate other than dGTP and terminal deoxytransferase. Even though the subsequent reactions were possible, quantitative performance was poor.

The methods of the present invention are methods in which the siRNA-dTT-polydG chimeric molecule modified as described above is converted into single-stranded DNA by performing reverse transcription reaction using polydC containing a tag sequence (tag-polydC) as the primer, and this single-stranded DNA is amplified and quantified by a chimeric nucleotide sequence-specific primer set within the siRNA nucleotide sequence. Quantification of full-length siRNA became possible by the above operation.

## Claims

1. A method for detecting an siRNA comprising a DNA overhang at the 3' end, which comprises the steps of:
(a) adding polydeoxyguanine (polydG) to the overhang of an siRNA;
(b) synthesizing a single-stranded DNA by performing a reverse transcription reaction using the molecule produced by the previous step as a template and a polydeoxycytosine (polydC) primer comprising a tag sequence at the 5' side;
(c) performing PCR using the single-stranded DNA as a template, a primer that anneals to the complementary strand of the tag sequence, and a primer that anneals to one of the strands of the siRNA; and
(d) detecting the PCR product.

2. The method of claim 1, wherein the primer that anneals to the complementary strand of the tag sequence in step (c) is the primer as defined in step (b).

3. A method for selectively amplifying a sequence constituting an siRNA comprising a DNA overhang at the 3' end, which comprises the steps of:
(a) adding polydeoxyguanine (polydG) to the overhang of an siRNA;
(b) synthesizing a single-stranded DNA by performing a reverse transcription reaction using the molecule produced by the previous step as a template and a polydeoxycytosine (polydC) primer comprising a tag sequence at the 5' side; and
(c) performing PCR using the single-stranded DNA as a template, a primer that anneals to the complementary strand of the tag sequence, and a primer that anneals to one of the strands of the siRNA.

4. The method of claim 3, wherein the primer that anneals to the complementary strand of the tag sequence in step (c) is the primer as defined in step (b).

5. The method of any one of claims 1 to 4, wherein the addition of polydeoxyguanine (polydG) is carried out by terminal deoxytransferase.

6. A method of monitoring the pharmacokinetics of an siRNA administered to an individual, which comprises the steps of:
(a) obtaining a test sample from a biological site where the amount of existing siRNA is to be measured; and
(b) detecting an siRNA of interest in the test sample by the method of claim 1, 2 or 5.

7. The method of claim 6, wherein the test sample is a blood or tissue sample.

## Patentansprüche

1. Verfahren zum Detektieren einer siRNA, umfassend einen DNA-Überhang am 3'-Ende, welches die folgenden Schritte umfasst:
(a) Zugeben von Polydesoxyguanin (polydG) zu dem Überhang einer siRNA;
(b) Synthetisieren einer einzelsträngigen DNA mittels Durchführen einer Umkehrtranskriptionsreaktion unter Verwendung des durch den vorherigen Schritt hergestellten Moleküls als eine Matrize und eines Polydesoxycytosin (polydC)-Primers, der eine Anhang-Sequenz an der 5'-Seite umfasst;
(c) Durchführen von PCR unter Verwendung der einzelsträngigen DNA als eine Matrize, eines Primers, der sich an den komplementären Strang der Anhang-Sequenz anlagert, und eines Primers, der sich an einen der Stränge der siRNA anlagert; und
(d) Detektieren des PCR-Produkts.

2. Verfahren nach Anspruch 1, wobei der Primer, der sich an den komplementären Strang der Anhang-Sequenz in Schritt (c) anlagert, der wie in Schritt (b) definierte Primer ist.

3. Verfahren, um selektiv eine Sequenz zu amplifizieren, die eine siRNA darstellt, die einen DNA-Überhang am 3'-Ende umfasst, welches die folgenden Schritte umfasst:
(a) Zugeben von Polydesoxyguanin (polydG) zu dem Überhang einer siRNA;
(b) Synthetisieren einer einzelsträngigen DNA mittels Durchführen einer Umkehrtranskriptionsreaktion unter Verwendung des durch den vorherigen Schritt hergestellten Moleküls als eine Matrize und eines Polydesoxycytosin (polydC)-Primers, der eine Anhang-Sequenz an der 5'-Seite umfasst;
(c) Durchführen von PCR unter Verwendung der einzelsträngigen DNA als eine Matrize, eines Primers, der sich an den komplementären Strang der Anhang-Sequenz anlagert, und eines Primers, der sich an einen der Stränge der siRNA anlagert.

4. Verfahren nach Anspruch 3, wobei der Primer, der sich an den komplementären Strang der Anhang-Sequenz in Schritt (c) anlagert, der wie in Schritt (b) definierte Primer ist.

5. Verfahren nach einem beliebigen der Ansprüche 1 bis 4, wobei die Zugabe von Polydesoxyguanin (polydG) mittels terminaler Desoxytransferase durchgeführt wird.

6. Verfahren zum Überwachen der Pharmakokinetik einer siRNA, verabreicht an ein Individuum, welches die folgenden Schritte umfasst:
(a) Erhalten einer Testprobe von einer biologischen Stelle, wo die Menge existierender siRNA zu messen ist; und
(b) Detektieren einer siRNA von Interesse in der Testprobe durch das Verfahren nach Anspruch 1, 2 oder 5.

7. Verfahren nach Anspruch 6, wobei die Testprobe eine Blut- oder Gewebeprobe ist.

## Revendications

1. Procédé de détection d'un siRNA comprenant une extrémité ADN simple brin sortante à l'extrémité 3', qui comprend les étapes suivantes :
(a) l'addition de polydésoxyguanine (polydG) à l'extrémité ADN simple brin sortante d'un siRNA ;
(b) la synthèse d'un ADN simple brin en réalisant une réaction de transcription inverse en utilisant la molécule produite par l'étape précédente comme matrice et une amorce de polydésoxycytosine (polydC) comprenant une séquence marqueur du côté 5' ;
(c) la réalisation d'une PCR en utilisant l'ADN simple brin comme matrice, une amorce qui s'hybride au brin complémentaire de la séquence marqueur, et une amorce qui s'hybride à l'un des brins du siRNA ; et
(d) la détection du produit de PCR.

2. Procédé selon la revendication 1, dans lequel l'amorce qui s'hybride au brin complémentaire de la séquence marqueur dans l'étape (c) est l'amorce telle que définie dans l'étape (b).

3. Procédé d'amplification sélective une séquence constituant un siRNA comprenant une extrémité ADN simple brin sortante à l'extrémité 3', qui comprend les étapes suivantes :
(a) l'addition de polydésoxyguanine (polydG) à l'extrémité ADN simple brin sortante d'un siRNA ;
(b) la synthèse d'un ADN simple brin en réalisant une réaction de transcription inverse en utilisant la molécule produite par l'étape précédente comme matrice et une amorce de polydésoxycytosine (polydC) comprenant une séquence marqueur du côté 5' ; et
(c) la réalisation d'une PCR en utilisant l'ADN simple brin comme matrice, une amorce qui s'hybride au brin complémentaire de la séquence marqueur, et une amorce qui s'hybride à l'un des brins du siRNA.

4. Procédé selon la revendication 3, dans lequel l'amorce qui s'hybride au brin complémentaire de la séquence marqueur dans l'étape (c) est l'amorce telle que définie dans l'étape (b).

5. Procédé selon l'une quelconque des revendications 1 à 4, dans lequel l'addition de polydésoxyguanine (polydG) est réalisée par une désoxytransférase terminale.

6. Procédé de contrôle de la pharmacocinétique d'un siRNA administré à un individu, qui comprend les étapes suivantes :
(a) l'obtention d'un échantillon à tester à partir du site biologique où la quantité de siRNA doit être mesurée ; et
(b) la détection d'un siRNA d'intérêt dans l'échantillon à tester par le procédé selon la revendication 1, 2 ou 5.

7. Procédé selon la revendication 6, dans lequel l'échantillon à tester est un échantillon de sang ou de tissu.
